# EUROPÄISCHE PATENTSCHRIFT

(11) **EP 3 207 981 B1**
(45) Veröffentlichungstag und Bekanntmachung des Hinweises auf die Patenterteilung: **05.09.2018**
(21) Anmeldenummer: 16156548.6
(22) Anmeldetag: 19.02.2016
(51) Int. Cl.: B01J 3/00, C07C 7/04, C07C 11/04

(54) **VERFAHREN UND ANLAGE ZUR GEWINNUNG EINES ETHYLENPRODUKTS IN ÜBERKRITISCHEM ZUSTAND**
METHOD AND PLANT FOR OBTAINING AN ETHYLENE PRODUCT IN SUPERCRITICAL STATE
PROCEDE ET INSTALLATION DESTINES A PRODUIRE UN PRODUIT D'ETHYLENE DANS UN ETAT SURCRITIQUE

(43) Veröffentlichungstag der Anmeldung: 23.08.2017
(73) Patentinhaber: Linde Aktiengesellschaft, 80331 München (DE)
(72) Erfinder: HÖFEL, Torben, 81543 München (DE); KAMANN, Martin, 82041 Oberhaching (DE); SCHÖLCH, Michael, 82319 Starnberg (DE); McCRACKEN, Sean, 82178 Puchheim (DE); KUNKEL, Josef, 82131 Gauting (DE); KRACKER-SEMLER, Gunther, 83629 Weyarn (DE)

(56) Entgegenhaltungen:
- WO-A1-2014/134703
- US-A- 2 813 920
- Heinz Zimmermann ET AL: "Ethylene" In: "Ullmann's Encyclopedia of Industrial Chemistry", 15. April 2009 (2009-04-15), Wiley-VCH Verlag GmbH & Co. KGaA, Weinheim, Germany, XP055007506, ISBN: 978-3-52-730673-2 DOI: 10.1002/14356007.a10_045.pub3, * Seite 45, rechte Spalte, Absatz "Ethylene Fractionation" - Seite 46, rechte Spalte erster Absatz *

## Beschreibung

Die Erfindung betrifft ein Verfahren zur Gewinnung eines Ethylenprodukts in überkritischem Zustand und eine entsprechende Anlage gemäß den jeweiligen Oberbegriffen der unabhängigen Patentansprüche.

### Stand der Technik

Verfahren und entsprechende Anlagen zur Gewinnung von Olefinen wie Ethylen durch Dampfspalten (engl. Steam Cracking) sind bekannt und z.B. im Artikel "Ethylene" in Ullmann's Encyclopedia of Industrial Chemistry, online seit 15. April 2009, DOI 10.1002/14356007.a10_045.pub3, beschrieben. Beim Dampfspalten werden zunächst Gasgemische erhalten, die einer Aufbereitung und, zur Trennung in Komponenten oder Komponentengruppen, Trennsequenzen unterworfen werden können. Beispielsweise sind sogenannte "Demethanizer First"-, "Deethanizer First"- und "Depropanizer First"-Verfahren aus dem Stand der Technik bekannt. US 2 813 920 offenbart ein Verfahren zur Herstellung von Ethylen. Auch das klassische Zielprodukt beim Dampfspalten, nämlich Ethylen, wird in entsprechenden Trennsequenzen von den anderen Komponenten abgetrennt. Zur Gewinnung von Ethylen wird in einer entsprechenden Trennsequenz hierzu eine Fraktion gebildet, die überwiegend oder ausschließlich Ethan und Ethylen enthält. Diese Fraktion wird in einer Destillationssäule, dem sogenannten C2-Splitter, in ein überwiegend oder ausschließlich Ethylen enthaltendes, gasförmiges Kopfprodukt und ein überwiegend oder ausschließlich Ethan enthaltendes, flüssiges Sumpfprodukt getrennt. Das Kopfprodukt des C2-Splitters wird teilweise in verflüssigter Form als Rücklauf auf den C2-Splitter zurückgeführt, ein weiterer Teil kann als flüssiges und/oder gasförmiges Ethylenprodukt bereitgestellt werden.

Die vorliegende Erfindung betrifft insbesondere die Verwendung von sogenannten Niederdruck-(LP-)C2-Splittern, die auf einem Druckniveau von typischerweise ca. 8 bis 9 bar betrieben werden. Die Verwendung solcher Niederdruck-C2-Splitter besitzt gegenüber der Verwendung von Hochdruck-(HP-)C2-Splittern Vorteile in Hinblick auf Investitionskosten und Energieverbrauch. Niederdruck-C2-Splitter sind in dem erwähnten Artikel "Ethylene" in Ullmann's Encyclopedia of Industrial Chemistry im Abschnitt "Heat-Pumped C2 Fractionation" auf Seite 47 beschrieben. Weitere Details bezüglich der Vorteile von Niederdruck-C2-Splittern sind dort angegeben.

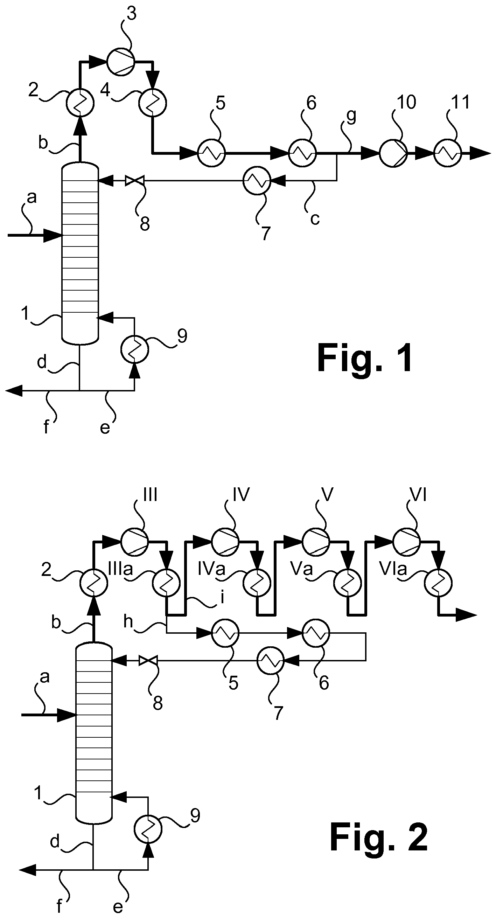

Bisweilen wird eine Bereitstellung des Ethylenprodukts in überkritischem Zustand gefordert. Hierzu kann das überwiegend oder ausschließlich Ethylen enthaltende, gasförmige Kopfprodukt vom Kopf des Niederdruck-C2-Splitters in einem nicht erfindungsgemäßen Verfahren des Standes der Technik, das auch unter Bezugnahme auf die Figur 1 erläutert wird, zunächst beispielsweise auf Umgebungstemperatur angewärmt, anschließend auf ein Druckniveau von beispielsweise mehr als 20 bar verdichtet, und durch Abkühlen auf diesem Druckniveau verflüssigt werden. Ein Teil des hierbei gewonnenen Verflüssigungsprodukts wird als der erwähnte Rücklauf auf den C2-Splitter verwendet. Der Rest des Verflüssigungsprodukts, oder auch nur ein Teil hiervon, wird mittels einer Pumpe vom flüssigem Zustand auf ein überkritisches Druckniveau befördert und auf dem überkritischen Druckniveau angewärmt.

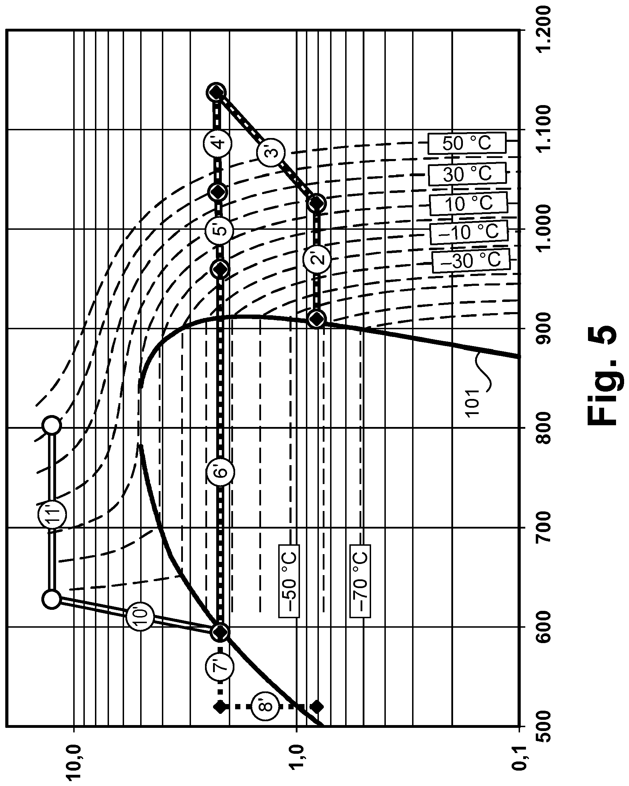

Wie insbesondere unter Bezugnahme auf die Figur 5 erläutert, weist ein entsprechendes Verfahren energetische Nachteile auf, da bei der Abkühlung eine große Energiemenge entzogen und für die Verdampfung auf dem überkritischen Druckniveau zusätzliche Energie zugeführt werden muss. Es besteht daher der Bedarf nach verbesserten Verfahren und Vorrichtungen zur Bereitstellung von gasförmigem Ethylen bei überkritischem Druck aus einem überwiegend oder ausschließlich Ethylen enthaltenden, gasförmigen Kopfprodukt vom Kopf eines Niederdruck-C2-Splitters.

### Offenbarung der Erfindung

Vor diesem Hintergrund schlägt die vorliegende Erfindung ein Verfahren zur Gewinnung eines Ethylenprodukts in überkritischem Zustand sowie eine entsprechende Anlage mit den Merkmalen der unabhängigen Patentansprüche vor. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Die vorliegende Anmeldung verwendet zur Charakterisierung von Drücken und Temperaturen die Begriffe "Druckniveau" und "Temperaturniveau", wodurch zum Ausdruck gebracht werden soll, dass Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere umfassen beispielsweise Druckniveaus auch unterschiedliche Drücke, die sich durch unvermeidliche Druckverluste ergeben. Entsprechendes gilt für Temperaturniveaus. Bei hier in bar angegebenen Druckniveaus handelt es sich um Absolutdrücke.

In Verfahren und Anlagen der eingangs erläuterten Art können zur Verdichtung mehrstufige Turboverdichter zum Einsatz kommen. Der mechanische Aufbau von Turboverdichtern ist dem Fachmann grundsätzlich bekannt. In einem Turboverdichter erfolgt die Verdichtung des zu verdichtenden Mediums mittels Turbinenschaufeln, die auf einem Turbinenrad oder direkt auf einer Welle angeordnet sind. Ein Turboverdichter bildet dabei eine bauliche Einheit, die jedoch bei einem mehrstufigen Verdichter mehrere Verdichterstufen aufweisen kann. Eine Verdichterstufe umfasst dabei in der Regel ein Turbinenrad oder eine entsprechende Anordnung von Turbinenschaufeln. Alle dieser Verdichterstufen können von einer gemeinsamen Welle angetrieben werden. Es kann jedoch auch vorgesehen sein, die Verdichterstufen gruppenweise mit unterschiedlichen Wellen anzutreiben, wobei die Wellen auch über Getriebe miteinander verbunden sein können.

Ein Wärmetauscher dient zur indirekten Übertragung von Wärme zwischen zumindest zwei z.B. im Gegenstrom zueinander geführten Fluidströmen. Ein Wärmetauscher zum Einsatz im Rahmen der vorliegenden Erfindung kann aus einem einzelnen oder mehreren parallel und/oder seriell verbundenen Wärmetauscherabschnitten gebildet sein, z.B. aus einem oder mehreren Plattenwärmetauscherblöcken.

Zur Auslegung und spezifischen Ausgestaltung von Destillationssäulen, wie sie im Rahmen der vorliegenden Anmeldung eingesetzt werden können, sei auf einschlägige Lehrbücher verwiesen (siehe beispielsweise K. Sattler, "Thermische Trennverfahren: Grundlagen, Auslegung, Apparate", 3. Auflage, Wiley-VCH, Weinheim 2001).

Bei einer Destillationssäule handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch (Trenneinsatz) zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert im oben erläuterten Sinne sind. Destillationssäulen sind aus dem Bereich der Trenntechnik hinlänglich bekannt. Typischerweise sind Destillationssäulen als zylindrische Metallbehälter ausgebildet, die mit Einbauten, beispielsweise Siebböden oder geordneten oder ungeordneten Packungen, ausgerüstet sind. Eine Destillationssäule zeichnet sich unter anderem dadurch aus, dass sich in ihrem unteren Bereich, auch als Sumpf bezeichnet, eine flüssige Fraktion abscheidet. Diese flüssige Fraktion, auch als Sumpfprodukt bezeichnet, wird in einer Destillationssäule mittels eines Sumpfverdampfers erwärmt, so dass kontinuierlich ein Teil des Sumpfprodukts verdampft und in der Destillationssäule gasförmig aufsteigt. Eine Destillationssäule ist ferner typischerweise mit einer Einrichtung versehen, in die zumindest ein Teil eines sich in einem oberen Bereich der Destillationssäule anreichernden Gasgemischs oder ein entsprechendes Reingas, auch als Kopfprodukt bezeichnet, eingespeist, dort verflüssigt und als flüssiger Rücklauf am Kopf der Destillationssäule aufgegeben wird.

### Vorteile der Erfindung

Die vorliegende Erfindung schlägt ein Verfahren zur Gewinnung eines Ethylenprodukts in überkritischem Zustand vor, bei dem ein überwiegend oder ausschließlich Ethylen und Ethan enthaltendes Gasgemisch in einer Destillationssäule, die auf einem Destillationsdruckniveau von 5 bis 15 bar, insbesondere 8 bis 10 bar, beispielsweise ca. 8,1 bar, betrieben wird, in einer überwiegend oder ausschließlich Ethylen enthaltendes Kopfprodukt und ein überwiegend oder ausschließlich Ethan enthaltendes Sumpfprodukt getrennt wird. Bei der zur Trennung des Gasgemischs verwendeten Destillationssäule handelt es sich daher um einen typischen Niederdruck-C2-Splitter. Zu weiteren Merkmalen eines derartigen Niederdruck-C2-Splitters sei auf die obigen Erläuterungen sowie die dort zitierte einschlägige Fachliteratur verwiesen.

Das in der Destillationssäule gebildete Kopfprodukt wird im gasförmigen Zustand vom Kopf der Destillationssäule abgezogen und zu einem ersten Anteil verflüssigt und als Rücklauf auf die Destillationssäule zurückgeführt. Zu einem zweiten Anteil wird das in gasförmigem Zustand vom Kopf der Destillationssäule abgezogene Kopfprodukt in einen überkritischen Zustand überführt und als das Ethylenprodukt verwendet.

Ist hier davon die Rede, dass ein erster Anteil und ein zweiter Anteil des Kopfprodukts auf die erläuterte Weise verwendet werden, sei hierunter verstanden, dass weitere Anteile eines entsprechenden Kopfprodukts anderweitig verwendet werden können; beispielsweise können diese in einem unterkritischen Zustand gasförmig oder flüssig als Ethylenprodukt bereitgestellt werden.

Wie bereits erläutert, ist in Verfahren des Standes der Technik, wie sie auch unter Bezugnahme auf die Figur 1 erläutert sind, vorgesehen, ein entsprechendes Kopfprodukt zur Bereitstellung eines Ethylenprodukts im überkritischen Zustand zunächst beispielsweise auf Umgebungstemperatur anzuwärmen, anschließend auf ein Druckniveau von beispielsweise mehr als 20 bar zu verdichten, und durch Abkühlen auf diesem Druckniveau zu verflüssigen. Im Stand der Technik wird das dabei gebildete Verflüssigungsprodukt in einem Anteil, der dem erfindungsgemäß gebildeten zweiten Anteil entspricht, aus dem flüssigem Zustand auf ein überkritisches Druckniveau befördert und auf diesem überkritischen Druckniveau angewärmt.

Im Gegensatz dazu schlägt die vorliegende Erfindung vor, zum Überführen des zweiten Anteils in den überkritischen Zustand eine mehrstufige Verdichtung von dem Destillationsdruckniveau, d.h. dem Druckniveau, auf dem die Destillationssäule betrieben wird, und auf dem das Kopfprodukt aus dieser Destillationssäule abgezogen wird, über mehrere Zwischendruckniveaus auf ein überkritisches Druckniveau zu verdichten. Der zweite Anteil wird in dieser mehrstufigen Verdichtung überwiegend oder ausschließlich direkt aus dem gasförmigen in den überkritischen Zustand überführt. Im Gegensatz zum Stand der Technik erfolgt also keine Verflüssigung und nachfolgende Anwärmung eines druckbeaufschlagten Verflüssigungsprodukts. Mit anderen Worten umfasst die Überführung des zweiten Anteils in den überkritischen Zustand im Rahmen der vorliegenden Erfindung keine Zwischenverflüssigung. Die vorliegende Erfindung weist dabei insbesondere energetische Vorteile gegenüber dem erläuterten Verfahren des Standes der Technik auf.

Weil gemäß dem Stand der Technik, ausgehend von dem erläuterten Druckniveau bei über 20 bar und einem entsprechend erhöhten Temperaturniveau (beispielsweise Umgebungstemperatur zuzüglich der Verdichtungswärme), eine Verflüssigung auch des zur Bereitstellung des Ethylenprodukts bei überkritischen Zustand verwendeten Anteils des von Kopf der Destillationssäule abgezogenen Kopfprodukts erfolgt, muss hierbei ein sehr großer Temperaturunterschied überwunden und damit eine große Wärmemenge entzogen werden. Dies ist anhand des in Figur 5 veranschaulichten Enthalpie-Druck-Diagramms nochmals erläutert. Nach der Druckbeaufschlagung im flüssigen Zustand muss anschließend wieder eine beträchtliche Energiemenge zugeführt werden, um eine Anwärmung auf Umgebungsbedingungen zur Bereitstellung des Ethylenprodukts zu bewirken.

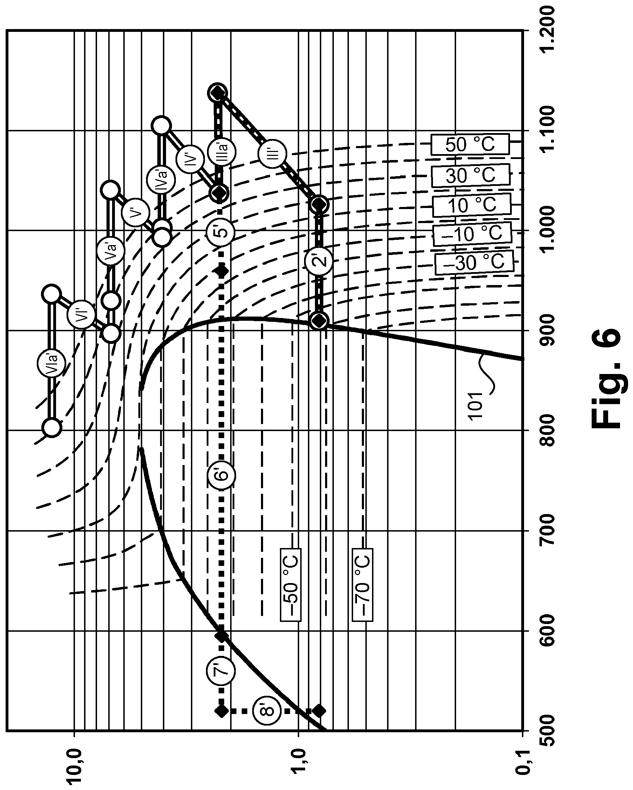

Im Gegensatz dazu ermöglicht es die vorliegende Erfindung, wie auch anhand des Enthalpie-Druck-Diagramms in Figur 6 näher erläutert, zur Bereitstellung des Ethylenprodukts in überkritischem Zustand auf entsprechend große Temperatursprünge zu verzichten. Lediglich der erste Anteil des vom Kopf der Destillationssäule abgezogenen Kopfprodukts, der als Rücklauf verwendet wird, muss einer entsprechend starken Abkühlung und Verflüssigung unterworfen werden. Der zweite Anteil wird mehrstufig verdichtet, wobei jeweils lediglich die Verdichtungswärme auf einem Temperaturnivau über Umgebungsbedingungen entzogen wird. Es ist dabei jeweils lediglich eine Abkühlung auf beispielsweise ca. 40 °C erforderlich, welche mit Kühlwasser bewerkstelligt werden kann. Durch den Verzicht auf eine Zwischenverflüssigung ermöglicht die vorliegende Erfindung die Einsparung entsprechend kostenintensiver, kälteresistenter Materialien.

Allgemein besteht im Stand der Technik das Vorurteil, eine Bereitstellung eines Druckprodukts in überkritischem Zustand sei besonders vorteilhaft, wenn, wie unter Bezugnahme auf den Stand der Technik erläutert, hierbei eine Zwischenverflüssigung und Druckbeaufschlagung des Verflüssigungsprodukts in flüssigem Zustand vorgenommen wird. Im Rahmen der vorliegenden Erfindung hat sich jedoch herausgestellt, dass die energetischen Vorteile einer stufenweisen Verdichtung im gasförmigen Zustand etwaige Nachteile deutlich überwiegen.

Im Rahmen der Erfindung wird, wie erwähnt, zum Überführen des zweiten Anteils in den überkritischen Zustand eine mehrstufige Verdichtung von dem Destillationsdruckniveau über mehrere Zwischendruckniveaus auf ein überkritisches Druckniveau vorgenommen. Der zweite Anteil wird dabei in der mehrstufigen Verdichtung überwiegend oder ausschließlich direkt aus dem gasförmigen in den überkritischen Zustand überführt.

Die erwähnten Zwischendruckniveaus umfassen dabei zumindest ein erstes Zwischendruckniveau, das bei 18 bis 25 bar, insbesondere 22 bis 23 bar, beispielsweise ca. 22,5 bar liegt. Eine Verdichtung auf ein derartiges Zwischendruckniveau ist besonders vorteilhaft, weil auf diesem Zwischendruckniveau der ebenfalls insoweit der mehrstufigen Verdichtung unterworfene erste Anteil, der als Rücklauf auf die Destillationssäule verwendet wird, ausgeschleust werden kann. Eine Verdichtung des ersten Anteils vor der Verflüssigung ist zweckmäßig, um die Verflüssigung bei ausreichend hohen Temperaturen bzw. mit den zur Verfügung stehenden Kältemitteln bewerkstelligen zu können.

Wie auch nachfolgend erläutert, kann ein derartiger erster Anteil nach einer entsprechenden Verdichtung, zusätzlich zur Abkühlung in weiteren Wärmetauschern, als Wärmeträger in einem Sumpfverdampfer der Destillationssäule verwendet werden. Auf diese Weise wird ein Wärmepumpeneffekt mit besonders vorteilhafter Energieausnutzung erzielt. Mit anderen Worten wird also der erste Anteil vorteilhafterweise in der mehrstufigen Verdichtung von dem Destillationsdruckniveau auf das erste Zwischendruckniveau verdichtet, anschließend verflüssigt und als der Rücklauf verwendet. Weiter ist, wie erwähnt, vorteilhaft, wenn die Destillationssäule mit einem Sumpfverdampfer betrieben wird, der unter Verwendung des ersten, auf das erste Zwischendruckniveau verdichteten Anteils beheizt wird.

Vorteilhafterweise umfasst die mehrstufige Verdichtung im Rahmen der vorliegenden Erfindung auch die Verdichtung von Kältemittel aus einem Kältemittelkreislauf, der wenigstens drei, auf unterschiedlichen Druckniveaus betriebene Teilkreisläufe umfasst. Ethylenkältemittel wird in entsprechenden Verfahren und Anlagen an unterschiedlicher Stelle verwendet, beispielsweise in einem Demethanizer oder anderen Trennschritten. Verfahren und Anlagen zur Bearbeitung von Gasgemischen, die durch Dampfspalten erzeugt werden, umfassen typischerweise mit Ethylenkältemittel betriebene Teilkreisläufe auf unterschiedlichen Druck- und damit Temperaturniveaus. Typischerweise sind dabei ein Niederdruckkältemittelkreislauf vorgesehen, der geringfügig oberhalb des atmosphärischen Druckniveaus oder leicht darunter, typischerweise bei 0,5 bis 1,5 bar, insbesondere 1,0 bis 1,1 bar, beispielsweise ca. 1,05 bar (nachfolgend als erstes Ausgangsdruckniveau bezeichnet), betrieben wird. Kältemittel in einem derartigen Niederdruckkältemittelkreislauf weist beispielsweise ein Temperaturniveau von ca. - 95 bis -100 °C auf. Ein Mitteldruckkältemittelkreislauf wird typischerweise auf einem Druckniveau von ca. 2,5 bis 3,5 bar, insbesondere 2,8 bis 3,2 bar, beispielsweise ca. 3 bar (nachfolgend als zweites Ausgangsdruckniveau bezeichnet), betrieben. Sein Kältemittel weist ein Temperaturniveau von typischerweise ca. - 75 bis - 85 °C auf. Schließlich ist ein sogenannter Hochdruckkältemittelkreislauf vorhanden, der auf einem Druckniveau von typischerweise ca. 5 bis 10 bar, insbesondere 8 bis 9 bar, beispielsweise ca. 8,1 bar, also dem Destillationsdruckniveau, betrieben wird. Das Kältemittel des Hochdruckkältemittelkreislaufs weist ein Temperaturniveau von typischerweise -55 bis -65 °C, insbesondere ca. -57 °C, auf.

Vorteilhafterweise werden daher in der mehrstufigen Verdichtung, die im Rahmen der Erfindung zum Einsatz kommt, ferner ein überwiegend oder ausschließlich Ethylen enthaltendes Kältemittel von mehreren Ausgangsdruckniveaus, die unterhalb des Destillationsdruckniveaus liegen, sowie von dem Destillationsdruckniveau auf das erste Zwischendruckniveau verdichtet. Auf diese Weise kann der erfindungsgemäß eingesetzte, mehrstufige Verdichter bzw. die entsprechende Verdichtung auch zur Bereitstellung von Kältemittel bzw. zur Beschickung der erwähnten Teilkreisläufe mit Kältemittel verwendet werden. Auf diese Weise wird ein offener Kältemittelkreislauf geschaffen, der besonders flexibel und energiesparend betrieben werden kann.

Im Rahmen der vorliegenden Erfindung ist zweckmäßig wenn die mehrstufige Verdichtung eine Verdichtung auf ein weiteres, d.h. zweites Zwischendruckniveau umfasst, das bei 35 bis 45, insbesondere 38 bis 42 bar, beispielsweise ca. 40,2 bar, liegt. Dies ist besonders vorteilhaft, weil in diesem Fall zum Verdichten von dem ersten auf das zweite Ausgangsdruckniveau eine erste, zum Verdichten von dem zweiten Ausgangsdruckniveau auf das Destillationsdruckniveau eine zweite, zum Verdichten von dem Destillationsdruckniveau auf das erste Zwischendruckniveau eine dritte, und zum Verdichten von dem ersten Zwischendruckniveau auf das zweite Zwischendruckniveau eine vierte Verdichterstufe bereitgestellt werden können, die insbesondere mittels einer ersten gemeinsamen Welle drehzahlgleich angetrieben werden können. Zum Antrieb dieser Verdichterstufen kann also eine gemeinsame Antriebsmaschine vorgesehen sein, weil die Verdichterlast auf die genannten Verdichterstufen im Wesentlichen gleichmäßig verteilt ist.

Vorteilhafterweise erfolgt danach der Verdichtung auf das zweite Zwischendruckniveau im Rahmen der vorliegenden Erfindung ferner eine Verdichtung auf ein drittes Zwischendruckniveau, das bei 60 bis 80 bar, insbesondere 65 bis 75 bar, beispielsweise ca. 70,4 bar, liegt, von welchem aus der zweite Anteil anschließend auf das überkritische Druckniveau von 100 bis 150 bar, insbesondere 120 bis 130 bar, beispielsweise ca. 125,6 bar, verdichtet wird.

Vorteilhafterweise werden zum Verdichten von dem zweiten auf das dritte Zwischendruckniveau eine fünfte und zum Verdichten von dem dritten Zwischendruckniveau auf das überkritische Druckniveau eine sechste Verdichterstufe verwendet. Die fünfte und sechste Verdichterstufe können vorteilhafterweise mittels einer zweiten gemeinsamen Welle drehzahlgleich angetrieben werden. Auf diese Weise wird durch den getrennten, jedoch jeweils gruppierten Antrieb der ersten bis vierten Verdichterstufe einerseits und der fünften und sechsten Verdichterstufe andererseits eine besonders gute Anpassbarkeit an die jeweiligen Verdichtungserfordernisse sichergestellt und regelungstechnische Vorteile erzielt. Insbesondere kann dabei vorgesehen sein, dass die ersten und die zweite gemeinsame Welle mittels eines Getriebes miteinander gekoppelt sind. Auf diese Weise können die ersten bis vierten Verdichterstufen einerseits und die fünften und sechsten Verdichterstufen anderseits drehzahlunterschiedlich betrieben werden.

Stromab der genannten Verdichterstufen erfolgt typischerweise eine Nachkühlung mittels geeigneter Nachkühler, die üblicherweise mit Kühlwasser betrieben werden. Stromab der ersten Verdichterstufe ist dabei nicht notwendigerweise ein entsprechender Nachkühler vorgesehen. Stromab der zweiten Verdichterstufe befindet sich zwar ein entsprechender Nachkühler, durch die gleichzeitige Zufuhr von Ethylenkältemittel auf dem entsprechenden Druckniveau wird der dritten Verdichterstufe jedoch Fluid mit einer Temperatur von typischerweise ca. 18 °C zugeführt. Den vierten, fünften und sechsten Verdichterstufen wird das Fluid jeweils mit typischerweise ca. 40 °C zugeführt, die es aufgrund der mit Wasser betriebenen Nachkühler erreicht. Anteile der jeweils verdichteten Fluide in den Verdichterstufen können auch zurückgeführt werden (sogenannte Kickbacks), insbesondere, um eine bessere Regelbarkeit der Verdichtung sicherzustellen.

Vorteilhafterweise kommt das erfindungsgemäße Verfahren im Rahmen eines Dampfspaltverfahrens zum Einsatz, d.h. das überwiegend ausschließlich Ethylen und Ethan enthaltende Gasgemisch wird unter Verwendung eines Spaltgases eines Dampfspaltverfahrens gebildet. Wie erwähnt, sind zur Bildung einer entsprechenden Gasgemischs unterschiedliche Verfahren aus dem Stand der Technik bekannt.

Die vorliegende Erfindung bezieht sich auch auf eine Anlage zur Gewinnung eines Ethylenprodukts in überkritischem Zustand, mit einer Destillationssäule, die dafür eingerichtet ist, ein überwiegend oder ausschließlich Ethylen und Ethan enthaltendes Gasgemisch auf einem Destillationsdruckniveau von 5 bis 15 bar in ein überwiegend oder ausschließlich Ethylen enthaltendes Kopfprodukt und ein überwiegend oder ausschließlich Ethan enthaltendes Sumpfprodukt zu trennen, wobei Mittel vorgesehen sind, die dafür eingerichtet sind, das Kopfprodukt in gasförmigem Zustand vom Kopf der Destillationssäule abzuziehen und zu einem ersten Anteil zu verflüssigen und als Rücklauf auf die Destillationssäule zurückzuführen und zu einem zweiten Anteil in einen überkritischen Zustand zu überführen und als das Ethylenprodukt zu verwenden. Erfindungsgemäß ist ein mehrstufiger Verdichter vorgesehen, der zum Überführen des zweiten Anteils in den überkritischen Zustand durch Verdichten von dem Destillationsdruckniveau über mehrere Zwischendruckniveaus auf ein überkritisches Druckniveau eingerichtet ist, wobei der zweite Anteil in dem mehrstufigen Verdichter überwiegend oder ausschließlich aus dem gasförmigen in den überkritischen Zustand überführt wird.

Eine entsprechende Anlage ist vorteilhafterweise zur Durchführung eines Verfahrens eingerichtet, wie es zuvor im Detail erläutert wurde und weist hierzu entsprechende Mittel auf. Auf die bezüglich des Verfahrens erläuterten Merkmale und Vorteile sei daher ausdrücklich verwiesen.

Die Erfindung wird nachfolgend unter Bezugnahme auf die beigefügten Zeichnungen näher erläutert, welche Aspekte der vorliegenden Erfindung gegenüber nicht erfindungsgemäßen Aspekten veranschaulichen.

### Kurze Beschreibung der Zeichnungen

Figur 1 veranschaulicht ein nicht erfindungsgemäßes Verfahren in Form eines schematischen Prozessflussdiagramms.
Figur 2 veranschaulicht ein Verfahren gemäß einer Ausführungsform der Erfindung in Form eines schematischen Prozessflussdiagramms.
Figur 3 veranschaulicht eine nicht erfindungsgemäße Verdichtung in Form eines schematischen Prozessflussdiagramms.
Figur 4 veranschaulicht eine Verdichtung gemäß einer Ausführungsform der Erfindung in Form eines schematischen Prozessflussdiagramms.
Figur 5 zeigt ein Enthalpie-Druck-Diagramm des in Figur 1 veranschaulichten, nicht erfindungsgemäßen Verfahrens.
Figur 6 zeigt ein Enthalpie-Druck-Diagramm des in Figur 2 veranschaulichten Verfahrens gemäß einer Ausführungsform der Erfindung.

In den Figuren sind einander entsprechende Elemente mit identischen Bezugszeichen angegeben und werden der Übersichtlichkeit halber nicht wiederholt erläutert.

In Figur 1 ist ein nicht erfindungsgemäßes Verfahren zur Gewinnung eines Ethylenprodukts in Form eines schematischen Prozessflussdiagramms veranschaulicht.

Das Verfahren umfasst die Verwendung einer Destillationssäule 1, die auf einem Druckniveau von 5 bis 15 bar, insbesondere 8 bis 10 bar, beispielsweise ca. 8,1 bar, betrieben wird, also dem mehrfach erwähnten Destillationsdruckniveau. Es handelt sich also um einen eingangs erläuterten Niederdruck-C2-Splitter. Der Destillationssäule 1 wird ein überwiegend oder ausschließlich Ethylen und Ethan enthaltendes Gasgemisch in Form eines Stroms a in geeigneter Höhe zugeführt. In der Destillationssäule 1 wird das in Form des Stroms a zugeführte Gasgemisch in ein überwiegend oder ausschließlich Ethylen enthaltendes Kopfprodukt und ein überwiegend oder ausschließlich Ethan enthaltendes Sumpfprodukt getrennt.

Das Kopfprodukt wird dabei in gasförmigen Zustand in Form eines Stroms b vom Kopf der Destillationssäule 1 abgezogen, in einem Wärmetauscher 2 auf beispielsweise Umgebungstemperatur angewärmt und in einem Verdichter 3 auf ein Druckniveau von mehr als 20 bar, beispielsweise 22,5 bar, verdichtet. Nach der Verdichtung in dem Verdichter 3 wird das Gasgemisch des Stroms b in Wärmetauschern 4 bis 6 abgekühlt und verflüssigt. Nach der Abkühlung in dem Wärmetauscher 6 wird ein Teil des Fluids des Stroms b in Form eines Stroms c einer weiteren Abkühlung in einem Wärmetauscher 7 zugeführt. Anschließend erfolgt eine Entspannung des Fluids des Stroms c in einem Ventil 8 auf das Druckniveau der Destillationssäule 1. Das Fluid des Stroms c wird am Kopf der Destillationssäule 1 als Rücklauf aufgegeben.

Aus dem Sumpf der Destillationssäule 1 wird das dort anfallende Sumpfprodukt in Form eines Stroms d abgezogen. Das Sumpfprodukt wird zu einem Teil in Form eines Stroms e durch einen Sumpfverdampfer 9 geführt, dort aufgekocht, und in die Destillationssäule 1 zurückgeführt, wo es gasförmig aufsteigt. Insbesondere können der Wärmetauscher 6 und der Wärmetauscher 9 auch thermisch gekoppelt sein bzw. in Form eines gemeinsamen Wärmetauschers ausgebildet sein. Auf diese Weise wird, wie zuvor erläutert, ein Wärmepumpeneffekt erzielt. Ein weiterer Anteil des Sumpfprodukts des Stroms d wird in Form des Stroms f ausgeführt. Da der Strom f überwiegend oder ausschließlich Ethan enthält, kann er beispielsweise in eine vorgelagerte Dampfspalteinrichtung zugeführt werden.

Der Strom g, der das nicht in Form des Stroms c auf die Destillationssäule 1 zurückgeführte Kopfprodukt des Stroms b und damit überwiegend oder ausschließlich Ethylen umfasst, wird in dem nicht erfindungsgemäßen Verfahren mittels einer Pumpe 10 in flüssigem Zustand druckbeaufschlagt. Die Druckerhöhung erfolgt daher bis auf ein überkritisches Druckniveau. Ausgehend vom flüssigen Zustand wird der Strom g zur Bereitstellung des Ethylenprodukts im überkritischen Zustand in einem weiteren Wärmetauscher 11 erwärmt.

In Figur 2 ist ein Verfahren zur Gewinnung eines Ethylenprodukts in überkritischem Zustand gemäß einer besonders bevorzugten Ausführungsform der Erfindung, ebenfalls in Form eines schematischen Prozessflussdiagramms, veranschaulicht. Elemente, die dabei jenen des nicht erfindungsgemäßen Verfahrens gemäß Figur 1 entsprechen, sind mit identischen Bezugszeichen angegeben und werden, wie erwähnt, der Übersichtlichkeit halber nicht wiederholt erläutert.

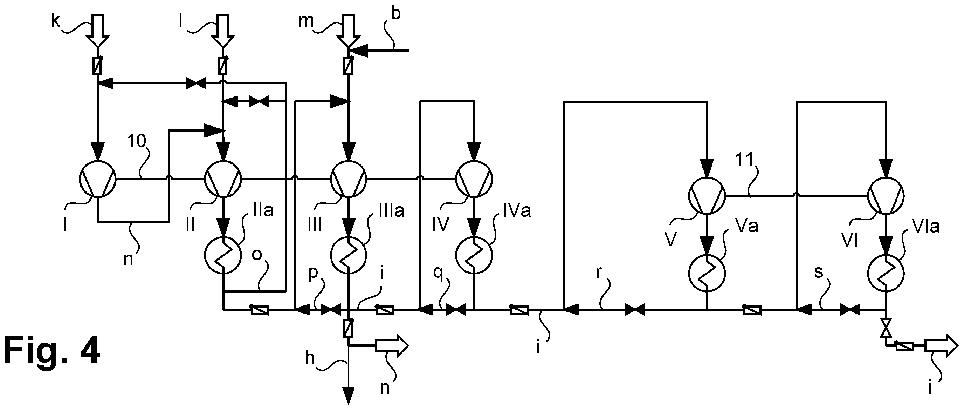

Auch im Rahmen des in Figur 2 veranschaulichten Verfahrens gemäß einer Ausführungsform der Erfindung wird der Strom b in einem Wärmetauscher 2 überhitzt. Nach der Anwärmung wird der Strom b dann jedoch in Verdichterstufen eines mehrstufigen Verdichters, die hier besseren Vergleichbarkeit mit der Figur 4 halber mit III bis VI bezeichnet sind, verdichtet. Stromab der Verdichtung erfolgt jeweils eine Nachkühlung in mit lila bis VIa bezeichneten Nachkühlern. Stromab der Verdichtung in der Verdichterstufe III wird ein Strom h abgezweigt, der grundsätzlich dem Strom c der Figur 1 entspricht und als Rücklauf auf die Destillationssäule 1 verwendet wird. Dieser Strom h wird in den Wärmetauschern 5, 6 und 7 abgekühlt und verflüssigt, wobei auch hier der Wärmetauscher 6 mit dem Wärmtauscher 9 thermisch gekoppelt bzw. in Form eines gemeinsamen Wärmetauschers ausgebildet sein kann. Nach entsprechender Abkühlung und Verflüssigung wird der Strom h über das Ventil 8 auf die Destillationssäule zurückgespeist und dort als Rücklauf verwendet.

In den Verdichterstufen III bis VI wird nicht in Form des Stroms h abgezweigter Rest, hier mit i bezeichnet, auf ein überkritisches Druckniveau verdichtet, wobei keine Zwischenverflüssigung erfolgt. Die Verdichterstufe III verdichtet dabei den Strom h von dem oben erläuterten Destillationsdruckniveau auf das erste Zwischendruckniveau, die Verdichterstufe IV verdichtet den Strom h von den ersten Zwischendruckniveau auf das zweite Zwischendruckniveau, die Verdichterstufe V verdichtet den Strom h von dem zweiten Zwischendruckniveau auf das dritte Zwischendruckniveau und die Verdichterstufe VI verdichtet den Strom h von dem dritten Zwischendruckniveau auf das überkritische Druckniveau. Die Druckniveaus wurden zuvor erläutert.

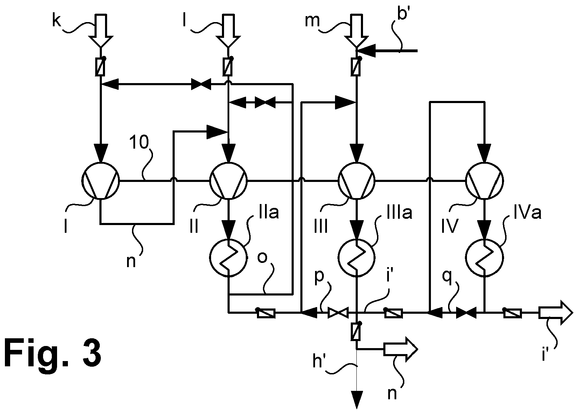

In Figur 3 ist eine Verdichtung gemäß einer nicht erfindungsgemäßen Ausführungsform schematisch in Form eines Prozessflussdiagramms veranschaulicht. Hierbei sind Verdichterstufen eines mehrstufigen Verdichters mit I bis IV veranschaulicht, wobei die Verdichterstufen III und IV der in Figur 3 gezeigten Verdichtung im wesentlichen den Verdichterstufen III und IV der in Figur 2 gezeigten Verdichtung entsprechen, hier jedoch die Verdichterstufen V und VI fehlen. Diese sind in der nachfolgenden Figur 4 veranschaulicht, die eine Verdichtung gemäß einer Ausführungsform der Erfindung veranschaulicht. Die in Figur 3 gezeigte Verdichtung kann in solchen Fällen in Verbindung mit einem Niederdruck-C2-Splitter zum Einsatz kommen, in welchen keine Verdichtung auf ein überkritisches Druckniveau und damit keine Bereitstellung eines Ethylenprodukts in überkritischen Zustand gefordert wird. In einer entsprechenden Verdichtung kann, je nach gefordertem Druckniveau, auch auf die vierte Verdichtungsstufe IV verzichtet werden. In diesem Fall wird das Ethylenprodukt auf dem druckseitigen Druckniveau der dritten Verdichtungsstufe III abgegeben.

Die in Figur 3 veranschaulichte Verdichtung ist zudem in einem Ethylenkältemittelkreislauf mit drei Teilkreisläufen eingebunden, wobei Kältemittelströme mit k, I und m bezeichnet sind. Wie erwähnt, erfolgt in der Verdichtung gemäß Figur 3 keine Bereitstellung eines Ethylenprodukts im überkritischen Zustand. Auch hier kann jedoch ein Kopfprodukt vom Kopf einer Destillationssäule 1, wie sie in Figur 2 gezeigt ist, eingespeist werden. Ein entsprechender Strom ist der besseren Unterscheidbarkeit halber hier mit b' bezeichnet. Der Strom k stellt Niederdruckkältemittel dar, das auf dem ersten Ausgangsdruckniveau und einem Temperaturniveau von ca. -95 bis -100 °C bereitgestellt wird. Der Strom I bezeichnet Mitteldruckkältemittel, das auf dem zweiten Ausgangsdruckniveau und einem Temperaturniveau von ca. 75 bis -85 °C bereitgestellt wird. Der Strom m bezeichnet Hochdruckkältemittel, das auf dem Destillationsdruckniveau und einem Temperaturniveau von ca. -55 bis -65 °C bereitgestellt wird. Die entsprechenden Ströme werden, wie in Figur 3 veranschaulicht, den Verdichterstufen I bis III ggf. nach Überhitzung zugeführt. Die Überführung des Kältemittels von der Verdichterstufe I zur Verdichterstufe II erfolgt direkt in der Maschine ohne Zwischenkühlung und ist hier in Form eines punktiert dargestellten Stroms n veranschaulicht.

Die Verdichterstufen I bis IV können über eine gemeinsame Welle, hier mit 10 bezeichnet, miteinander verbunden sein. Das in der Verdichterstufe II verdichtete Fluid wird in dem Wärmetauscher IIa abgekühlt und zumindest zu einem überwiegenden Teil anschließend der Verdichterstufe III zugeführt. Ein gewisser Anteil kann auch in Form eines sogenannten Kickbacks zur Verdichterstufe I zurückgeführt werden. Entsprechend wird das Fluid in der Verdichterstufe III verdichtet und anschließend in dem Wärmetauscher lila abgekühlt. Wiederum kann ein Teil vor die Verdichterstufe III zurückgeführt werden, wie mit dem Strom p veranschaulicht. Ein weiterer Teil kann, wie hier in Form des Stroms h' veranschaulicht, als Rücklauf auf die Destillationssäule verwendet werden. Ein weiterer Strom, wie hier mit n veranschaulicht, wird auf dem ersten Zwischendruckniveau, auf das die Verdichterstufe III das Fluid verdichtet, in den Kältemittelkreislauf zurückgeführt. Wie hier nicht veranschaulicht, kann das Kältemittel des Stroms n anschließend auf die zuvor erläuterten Druckniveaus der Teilkreisläufe bzw. Ströme k, I und m entspannt werden. Der verbleibende Anteil, wie hier in Form des Stroms i' veranschaulicht, wird der vierten Verdichterstufe IV zugeführt, dort auf das zweite Zwischendruckniveau verdichtet und anschließend in einem Nachkühler IVa abgekühlt. Wiederum kann ein Anteil, wie hier in Form des Stroms q veranschaulicht, vor die Verdichterstufe IV zurückgeführt werden. Der Rest, wie hier in Form des Stroms i' veranschaulicht, kann als Ethylenprodukt auf einem unterkritischen Druckniveau bereitgestellt werden.

Im Gegensatz dazu umfasst die in Figur 4 veranschaulichte Verdichtung gemäß einer Ausführungsform der Erfindung die zwei weitere Verdichterstufen V und VI, die bereits in Figur 2 dargestellt sind. Diese werden grundsätzlich vergleichbar der Verdichterstufe IV betrieben, verdichten jedoch das Fluid des Stroms, der hier dem Strom i aus Figur 2 entspricht und daher hier identisch bezeichnet ist, weiter auf nochmals höhere Druckniveaus. Die Verdichterstufe V verdichtet das Fluid dabei auf das dritte Zwischendruckniveau, in dem Nachkühler Va wird das Fluid dabei auf eine Temperatur von beispielsweise ca. 40 °C abgekühlt. Die Verdichterstufe VI verdichtet das Fluid schließlich auf das überkritische Druckniveau (auch das dritte Zwischendruckniveau kann schon überkritisch sein), wobei auch hier im Nachkühler VIa eine Abkühlung auf ein Temperaturniveau von beispielsweise ca. 40 °C erfolgt.

Die Vorteile der vorliegenden Erfindung gegenüber dem nicht erfindungsgemäßen Verfahren werden nachfolgend unter Bezugnahme auf die in den Figuren 5 und 6 veranschaulichten Enthalpie-Druck-Diagramme erläutert. In diesen jeweils ein Druck in MPa auf der Ordinate gegenüber einer Enthalpie in kJ/kg auf der Abszisse aufgetragen. Mit 101 (fette, durchgezogene Linie) ist dabei jeweils die Zweiphasenlinie der Enthalpie-Druck-Diagramme veranschaulicht. Die Isothermenlinien sind (teilweise) mit ihren jeweiligen Temperaturen bezeichnet.

Wie erwähnt, veranschaulicht Figur 5 das Enthalpie-Druck-Diagramm des in Figur 1 veranschaulichten, nicht erfindungsgemäßen Verfahrens. Zur besseren Anschaulichkeit sind dabei die durch die in Figur 1 gezeigten Apparate bewirkten Zustandsänderungen in dem Enthalpie-Druck-Diagramm mit den entsprechenden Bezugszeichen veranschaulicht, die jeweils zur Abgrenzung mit einem Hochkomma, versehen sind. Die Behandlung des Ethylenprodukts (Strom g in Figur 1) ist in Form einer Doppellinie, die Behandlung des Rücklaufs auf die Destillationssäule (Strom c in Figur 1) in Form einer fetten gepunkteten Linie veranschaulicht. Wo diese Ströme gemeinsam verlaufen (Strom b in Figur 1) sind die Doppellinie und die gepunktete Linie überlagert dargestellt.

Durch die Erwärmung in dem Wärmetauscher 2 gemäß Figur 1, hier mit 2' bezeichnet, nimmt das vom Kopf der Destillationssäule abgezogene Fluid des Stroms b Energie auf. Anschließend erfolgt, wie hier mit 3' veranschaulicht, eine Verdichtung in dem Verdichter 3. Hierzu erfährt das Fluid einerseits eine Druckerhöhung und andererseits eine Erwärmung aufgrund der Aufnahme von Verdichtungswärme. Anschließend wird das Fluid des Stroms b in den Wärmetauschern 4 bis 6 abgekühlt und dabei verflüssigt. Dies in dem Enthalpie-Druck-Diagramm der Figur 5 mit 4' bis 6' veranschaulicht. Wie in dem Enthalpie-Druck-Diagramm mit 7' und 8' bezeichnet, wird das Fluid des Stroms c anschließend einer weiteren Abkühlung in den Wärmetauscher 7 unterworfen und anschließend in dem Ventil 8 entspannt. Hierdurch gelangt das Fluid in das Zweiphasengebiet und wird entsprechend zweiphasig in die Destillationssäule 1 eingespeist. Der Strom g, der nicht auf die Destillationssäule zurückgeführt wird, wird hingegen in der Pumpe 10, wie in dem Enthalpie-Druck-Diagramm der Figur 5 mit 10' bezeichnet, flüssig druckbeaufschlagt und erfährt hierdurch eine entsprechende Druck- und Temperaturerhöhung, wobei eine Verdichtung auf die zuvor erläuterten überkritischen Werte vorgenommen wird. Es folgt eine Erwärmung des überkritischen Fluids in dem Wärmetauscher 11.

Wie aus dem Enthalpie-Druck-Diagramm in Figur 5 ersichtlich, muss hierbei durch die Abkühlung in den Wärmetauschern 4 bis 6 ein deutlicher Temperaturunterschied überwunden werden, dem Fluid des Stroms b wird also eine beträchtliche Energiemenge entzogen. Für die Erwärmung des Fluids des Stroms g im dem Wärmetauscher 11 wird anschließend eine beträchtliche Energiemenge erneut zugeführt. Dies erweist sich, wie erfindungsgemäß festgestellt wurde, als energetisch nicht vorteilhaft.

Wie erwähnt, veranschaulicht Figur 6 das Enthalpie-Druck-Diagramm des Verfahrens gemäß der Ausführungsform, die in Figur 2 veranschaulicht ist. Auch hier sind die den in Figur 2 gezeigten Apparaten entsprechenden Zustandsänderungen mit Bezugszeichen mit Hochkommas angegeben. Die Behandlung des Ethylenprodukts (Strom i in Figur 2) ist in Form einer Doppellinie, die Behandlung des Rücklaufs auf die Destillationssäule (Strom h in Figur 2) in Form einer fetten gepunkteten Linie veranschaulicht. Wo diese Ströme gemeinsam verlaufen (Strom b in Figur 2) sind auch hier die Doppellinie und die gepunktete Linie überlagert dargestellt.

Die Erwärmung in dem Wärmetauscher 2 entspricht, in dem Enthalpie-Druck-Diagramm 6 mit 2' bezeichnet, entspricht dabei zunächst der Erwärmung in dem nicht erfindungsgemäßen Verfahren gemäß Figur 2. Entsprechendes gilt auch für die Verdichtung in der Verdichtungsstufe III, in Figur 5 mit III' bezeichnet. Nach dieser Verdichtung erfolgt, wie in Figur 6 mit IIIa' bezeichnet, eine Abkühlung. Diese erfolgt beispielsweise auf ein Temperaturniveau von ca. 40 °C, wie hier aus der entsprechenden Isotherme ersichtlich. Wie mit der in Figur 6 mit einer gestrichelten Linie bezeichnet, wird jedoch nun nur ein Teil des Fluids des Stroms b, nämlich das Fluid des Stroms h, verflüssigt. Hierbei kommen die Wärmetauscher 5 bis 7 zum Einsatz, die entsprechenden Abkühlvorgänge sind in dem Enthalpie-Druck-Diagramm der Figur 6 mit 5' bis 7' veranschaulicht. Entsprechendes gilt auch für die mit 8' veranschaulichte Entspannung in dem Entspannungsventil 8.

Der verbleibende Rest in Form des Stroms i wird nun in der Verdichterstufe IV verdichtet, in Figur 6 mit IV' veranschaulicht, anschließend in dem Nachkühler VIa abgekühlt, in Figur 6 mit VIa' veranschaulicht, usw. Die weiteren Verdichtungs- und Abkühlungsstufen sind unmittelbar aus Figur 6 ersichtlich. Nach der Verdichtung in der Verdichterstufe VI und der Abkühlung in dem Nachkühler VIa liegt das Ethylenprodukt auf einem überkritischen Druckniveau von typischerweise ca. 125,6 bar und auf einem Temperaturniveau von beispielsweise ca. 40 bar vor.

## Patentansprüche

1. Verfahren zur Gewinnung eines Ethylenprodukts in überkritischem Zustand, bei dem ein überwiegend oder ausschließlich Ethylen und Ethan enthaltendes Gasgemisch in einer Destillationssäule (1), die auf einem Destillationsdruckniveau von 5 bis 15 bar betrieben wird, in ein überwiegend oder ausschließlich Ethylen enthaltendes Kopfprodukt und ein überwiegend oder ausschließlich Ethan enthaltendes Sumpfprodukt getrennt wird, wobei das Kopfprodukt in gasförmigem Zustand vom Kopf der Destillationssäule (1) abgezogen und zu einem ersten Anteil verflüssigt und als Rücklauf auf die Destillationssäule (1) zurückgeführt und zu einem zweiten Anteil in einen überkritischen Zustand überführt und als das Ethylenprodukt verwendet wird, **dadurch gekennzeichnet, dass** zum Überführen des zweiten Anteils in den überkritischen Zustand eine mehrstufige Verdichtung von dem Destillationsdruckniveau über mehrere Zwischendruckniveaus auf ein überkritisches Druckniveau vorgenommen wird, wobei der zweite Anteil in der mehrstufigen Verdichtung überwiegend oder ausschließlich direkt aus dem gasförmigen in den überkritischen Zustand überführt wird.

2. Verfahren nach Anspruch 1, bei dem die Zwischendruckniveaus ein erstes Zwischendruckniveau, das bei 18 bis 25 bar liegt, umfassen.

3. Verfahren nach Anspruch 2, bei dem der erste Anteil in der mehrstufigen Verdichtung von dem Destillationsdruckniveau auf das erste Zwischendruckniveau verdichtet, anschließend verflüssigt und als der Rücklauf verwendet wird.

4. Verfahren nach Anspruch 3, bei dem die Destillationssäule (1) mit einem Sumpfverdampfer betrieben wird, der unter Verwendung des ersten, auf das erste Zwischendruckniveau verdichteten Anteils beheizt wird.

5. Verfahren nach einem der Ansprüche 2 bis 4, bei dem in der mehrstufigen Verdichtung ferner ein überwiegend oder ausschließlich Ethylen enthaltendes Kältemittel von mehreren Ausgangsdruckniveaus, die unterhalb des Destillationsdruckniveaus liegen, sowie von dem Destillationsdruckniveau auf das erste Zwischendruckniveau verdichtet wird.

6. Verfahren nach Anspruch 5, bei dem die Ausgangsdruckniveaus ein erstes Ausgangsdruckniveau, das bei 0,5 bis 1,5 bar liegt, und ein zweites Ausgangsdruckniveau, das bei 2 bis 4 bar liegt, umfassen.

7. Verfahren nach Anspruch 6, bei dem die Zwischendruckniveaus ferner ein zweites Zwischendruckniveau, das bei 35 bis 45 bar liegt, umfassen.

8. Verfahren nach Anspruch 7, bei dem zum Verdichten von dem ersten auf das zweite Ausgangsdruckniveau eine erste (I), zum Verdichten von dem zweiten Ausgangsdruckniveau auf das Destillationsdruckniveau eine zweite (II), zum Verdichten von dem Destillationsdruckniveau auf das erste Zwischendruckniveau eine dritte (III), und zum Verdichten von dem ersten Zwischendruckniveau auf das zweite Zwischendruckniveau eine vierte Verdichterstufe (IV) verwendet werden, wobei die erste bis vierte Verdichterstufe (I-IV) mittels einer ersten gemeinsamen Welle (10) drehzahlgleich angetrieben werden.

9. Verfahren nach Anspruch 8, bei dem die Zwischendruckniveaus ferner ein drittes Zwischendruckniveau, das bei 60 bis 80 bar liegt, umfassen, und bei dem das überkritische Druckniveau bei 100 bis 150 bar liegt.

10. Verfahren nach Anspruch 9, bei dem zum Verdichten von dem zweiten auf das dritte Zwischendruckniveau eine fünfte (V) und zum Verdichten von dem dritten Zwischendruckniveau auf das überkritische Druckniveau eine sechste Verdichterstufe (VI) verwendet werden, wobei die fünfte und die sechste Verdichterstufe (V, VI) mittels einer zweiten gemeinsamen Welle (11) drehzahlgleich angetrieben werden.

11. Verfahren nach Anspruch 10, bei dem die erste und die zweite gemeinsame Welle mittels eines Getriebes miteinander gekoppelt sind.

12. Verfahren nach einem der vorstehenden Ansprüche, bei dem das überwiegend oder ausschließlich Ethylen und Ethan enthaltende Gasgemisch unter Verwendung eines Spaltgases eines Dampfspaltverfahrens gebildet wird.

13. Anlage zur Gewinnung eines Ethylenprodukts in überkritischem Zustand, mit einer Destillationssäule (1), die dafür eingerichtet ist, ein überwiegend oder ausschließlich Ethylen und Ethan enthaltendes Gasgemisch auf einem Destillationsdruckniveau von 5 bis 15 bar in ein überwiegend oder ausschließlich Ethylen enthaltendes Kopfprodukt und ein überwiegend oder ausschließlich Ethan enthaltendes Sumpfprodukt zu trennen, wobei Mittel vorgesehen sind, die dafür eingerichtet sind, das Kopfprodukt in gasförmigem Zustand vom Kopf der Destillationssäule (1) abzuziehen und zu einem ersten Anteil zu verflüssigen und als Rücklauf auf die Destillationssäule (1) zurückzuführen und zu einem zweiten Anteil in einen überkritischen Zustand zu überführen und als das Ethylenprodukt zu verwenden, **gekennzeichnet durch** einen mehrstufigen Verdichter (I-VI), der zum Überführen des zweiten Anteils in den überkritischen Zustand durch Verdichten von dem Destillationsdruckniveau über mehrere Zwischendruckniveaus auf ein überkritisches Druckniveau eingerichtet ist, wobei der zweite Anteil in dem mehrstufigen Verdichter (I-VI) überwiegend oder ausschließlich aus dem gasförmigen in den überkritischen Zustand überführt wird.

14. Anlage nach Anspruch 13, die zur Durchführung eines Verfahrens nach einem der Ansprüche 1 bis 12 ausgebildet ist und hierzu eingerichtete Mittel aufweist.

## Claims

1. Method for obtaining an ethylene product in a supercritical state, in which a gas mixture containing predominantly or exclusively ethylene and ethane is separated in a distillation column (1) operated at a distillation pressure level of 5 to 15 bar into a top product containing predominantly or exclusively ethylene and a bottom product containing predominantly or exclusively ethane, wherein the top product is drawn off from the top of the distillation column (1) in the gaseous state and a first fraction is liquefied and recycled as reflux to the distillation column (1) and a second fraction is converted to a supercritical state and used as the ethylene product, **characterized in that**, for converting the second fraction to the supercritical state, a multistage compression is performed from the distillation pressure level over multiple intermediate pressure levels to a supercritical pressure level, the second fraction being predominantly or exclusively converted to the supercritical state in the multistage compression directly from the gaseous state.

2. Method according to Claim 1, in which the intermediate pressure levels comprise a first intermediate pressure level that lies at 18 to 25 bar.

3. Method according to Claim 2, in which the first fraction is compressed in the multistage compression from the distillation pressure level to the first intermediate pressure level, subsequently liquefied and used as the reflux.

4. Method according to Claim 3, in which the distillation column (1) is operated with a bottom evaporator, which is heated by using the first fraction compressed to the first intermediate pressure level.

5. Method according to one of Claims 2 to 4, in which, in the multistage compression, furthermore, a refrigerant containing predominantly or exclusively ethylene is compressed from multiple starting pressure levels that lie below the distillation pressure level and from the distillation pressure level to the first intermediate pressure level.

6. Method according to Claim 5, in which the starting pressure levels comprise a first starting pressure level that lies at 0.5 to 1.5 bar and a second starting pressure level that lies at 2 to 4 bar.

7. Method according to Claim 6, in which the intermediate pressure levels also comprise a second intermediate pressure level that lies at 35 to 45 bar.

8. Method according to Claim 7, in which a first compressor stage (I) is used for compressing from the first starting pressure level to the second starting pressure level, a second compressor stage (II) is used for compressing from the second starting pressure level to the distillation pressure level, a third compressor stage (III) is used for compressing from the distillation pressure level to the first intermediate pressure level, and a fourth compressor stage (IV) is used for compressing from the first intermediate pressure level to the second intermediate pressure level, the first to fourth compressor stages (I-IV) being driven at the same rotational speed by means of a first common shaft (10) .

9. Method according to Claim 8, in which the intermediate pressure levels also comprise a third intermediate pressure level that lies at 60 to 80 bar and in which the supercritical pressure level lies at 100 to 150 bar.

10. Method according to Claim 9, in which a fifth compressor stage (V) is used for compressing from the second intermediate pressure level to the third intermediate pressure level and a sixth compressor stage (VI) is used for compressing from the third intermediate pressure level to the supercritical pressure level, the fifth and sixth compressor stages (V, VI) being driven at the same rotational speed by means of a second common shaft (11).

11. Method according to Claim 10, in which the first and second common shafts are coupled to one another by means of a gear mechanism.

12. Method according to one of the preceding claims, in which the gas mixture containing predominantly or exclusively ethylene and ethane is formed by using a cracked gas of a steam cracking process.

13. Plant for obtaining an ethylene product in a supercritical state, with a distillation column (1) which is designed for separating a gas mixture containing predominantly or exclusively ethylene and ethane at a distillation pressure level of 5 to 15 bar into a top product containing predominantly or exclusively ethylene and a bottom product containing predominantly or exclusively ethane, wherein means which are designed for drawing off the top product from the top of the distillation column (1) in the gaseous state and liquefying a first fraction and recycling it as reflux to the distillation column (1) and for converting a second fraction to a supercritical state and using it as the ethylene product are provided, **characterized by** a multistage compressor (I-VI) which is designed for converting the second fraction to the supercritical state by compressing it from the distillation pressure level over multiple intermediate pressure levels to a supercritical pressure level, the second fraction being predominantly or exclusively converted to the supercritical state in the multistage compressor (I-VI) from the gaseous state.

14. Plant according to Claim 13, which is formed for carrying out a method according to one of Claims 1 to 12 and has means designed for this.

## Revendications

1. Procédé destiné à produire un produit d'éthylène dans un état supercritique, dans lequel on sépare un mélange gazeux contenant principalement ou exclusivement de l'éthylène et de l'éthane dans une colonne de distillation (1), qui fonctionne à un niveau de pression de distillation de 5 à 15 bar, en un produit de tête contenant principalement ou exclusivement de l'éthylène et un produit de pied contenant principalement ou exclusivement de l'éthane, dans lequel on extrait le produit de tête sous forme gazeuse à la tête de la colonne de distillation (1), on le liquéfie à raison d'une première part et on le renvoie en retour à la colonne de distillation (1) et on l'amène dans un état supercritique à raison d'une deuxième part et on l'utilise comme produit d'éthylène, **caractérisé en ce que** pour amener la deuxième part dans l'état supercritique on opère une compression en plusieurs étages à partir du niveau de pression de distillation par plusieurs niveaux de pression intermédiaires jusqu'à un niveau de pression supercritique, dans lequel on amène la deuxième part dans l'état supercritique dans la compression à plusieurs étages principalement ou exclusivement directement à partir de l'état gazeux.

2. Procédé selon la revendication 1, dans lequel les niveaux de pression intermédiaires comprennent un premier niveau de pression intermédiaire, qui est de 18 à 25 bar.

3. Procédé selon la revendication 2, dans lequel on comprime la première part dans la compression à plusieurs étages à partir du niveau de pression de distillation jusqu'au premier niveau de pression intermédiaire, puis on la liquéfie et on l'utilise comme retour.

4. Procédé selon la revendication 3, dans lequel on fait fonctionner la colonne de distillation (1) avec un évaporateur de pied, que l'on chauffe en utilisant la première part comprimée au premier niveau de pression intermédiaire.

5. Procédé selon l'une quelconque des revendications 2 à 4, dans lequel on comprime en outre dans la compression à plusieurs étages un agent réfrigérant contenant principalement ou exclusivement de l'éthylène à partir de plusieurs niveaux de pression initiaux, qui sont situés en dessous du niveau de pression de distillation, ainsi qu'à partir du niveau de pression de distillation, jusqu'au premier niveau de pression intermédiaire.

6. Procédé selon la revendication 5, dans lequel les niveaux de pression initiaux comprennent un premier niveau de pression initial, qui est de 0,5 à 1,5 bar, et un second niveau de pression initial, qui est de 2 à 4 bar.

7. Procédé selon la revendication 6, dans lequel les niveaux de pression intermédiaires comprennent en outre un deuxième niveau de pression intermédiaire, qui est de 35 à 45 bar.

8. Procédé selon la revendication 7, dans lequel on utilise pour la compression du premier au second niveau de pression initial un premier (I), pour la compression du second niveau de pression initial au niveau de pression de distillation un deuxième (II), pour la compression du niveau de pression de distillation au premier niveau de pression intermédiaire un troisième (III), et pour la compression du premier niveau de pression intermédiaire au deuxième niveau de pression intermédiaire un quatrième (IV) étage de compression, dans lequel on entraîne le premier au quatrième étage de compresseur (I-IV) à la même vitesse de rotation au moyen d'un premier arbre commun (10).

9. Procédé selon la revendication 8, dans lequel les niveaux de pression intermédiaires comprennent en outre un troisième niveau de pression intermédiaire, qui est de 60 à 80 bar, et dans lequel le niveau de pression supercritique est de 100 à 150 bar.

10. Procédé selon la revendication 9, dans lequel on utilise pour la compression du deuxième au troisième niveau de pression intermédiaire un cinquième (V) et pour la compression du troisième niveau de pression intermédiaire au niveau de pression supercritique un sixième (VI) étage de compresseur, dans lequel on entraîne le cinquième et le sixième étages de compresseur (V, VI) à la même vitesse de rotation au moyen d'un deuxième arbre commun (11).

11. Procédé selon la revendication 10, dans lequel le premier et le deuxième arbres communs sont couplés l'un à l'autre au moyen d'un engrenage.

12. Procédé selon l'une quelconque des revendications précédentes, dans lequel on forme le mélange gazeux contenant principalement ou exclusivement de l'éthylène et de l'éthane en utilisant un gaz de craquage d'un procédé de craquage à la vapeur.

13. Installation destinée à produire un produit d'éthylène dans un état supercritique, avec une colonne de distillation (1), qui est conçue pour séparer un mélange gazeux contenant principalement ou exclusivement de l'éthylène et de l'éthane à un niveau de pression de distillation de 5 à 15 bar en un produit de tête contenant principalement ou exclusivement de l'éthylène et un produit de pied contenant principalement ou exclusivement de l'éthane, dans laquelle il est prévu des moyens qui sont conçus pour extraire le produit de tête à l'état gazeux de la tête de la colonne de distillation (1) et pour le liquéfier à raison d'une première part et pour le renvoyer en retour à la colonne de distillation (1) et pour l'amener à raison d'une deuxième part dans un état supercritique et pour l'utiliser comme produit d'éthylène, **caractérisée par** un compresseur à plusieurs étages (I-VI), qui est conçu pour amener la deuxième part dans l'état supercritique par compression depuis le niveau de pression de distillation par plusieurs niveaux de pression intermédiaires à un niveau de pression supercritique, dans laquelle la deuxième part est amenée dans le compresseur à plusieurs étages (I-VI) principalement ou exclusivement de l'état gazeux à l'état supercritique.

14. Installation selon la revendication 13, qui est réalisée de façon à exécuter un procédé selon l'une quelconque des revendications 1 à 12 et qui présente des moyens conçus à cet effet.
